# EUROPEAN PATENT APPLICATION

(11) **EP 1 354 601 A1**
(43) Date of publication of application: **22.10.2003**
(21) Application number: 01273283.0
(22) Date of filing: 17.12.2001
(51) Int. Cl.: A61K 45/00, A61K 31/198, A61K 31/22, A61K 31/223, A61K 31/255, A61P 27/02, A61P 37/06, A61P 43/00, A23L 1/30

(54) **TRANSPLANTATION REJECTION INHIBITORS**

(30) Priority: 26.12.2000 JP 2000003947
(71) Applicant: Ajinomoto Co., Inc., Tokyo 104-8315 (JP)
(72) Inventor: HAMURO, Junji c/o Ctr Res. Lab. Ajinomoto Co.Inc., Kawasaki-shi, Kanagawa 210-0801 (JP); MURATA, Yukie c/o Ctr Res. Lab. Ajinomoto Co.Inc., Kawasaki-shi, Kanagawa 210-0801 (JP); YAMADA, Jun, Otokuni-gun, Kyoto 618-0091 (JP); SANO, Yoichiro, Kyoto-shi, Kyoto 604-0804 (JP); KINOSHITA, Shigeru, Osaka-shi, Osaka 545-0035 (JP)
(74) Representative: HOFFMANN - EITLE
(86) International application number: JP0111054
(87) International publication number: WO02056911

(57) **Abstract**

The present invention provides a transplant rejection suppressor usable as pharmaceuticals (oral preparations, eye drops, injections, infusions and the like) effective for suppressive treatment of human organ transplant rejection by detecting an oxidized and/or reduced glutathione content in macrophage cells using a body fluid/cell sample separated and collected from human beings to classify macrophages into oxidative macrophages and reductive macrophages different from each other in function, and artificially deviating the presence ratio thereof to oxidative macrophages with a specific low-molecular compound or artificially removing reductive macrophages, and further provides a use of the active ingredient for this purpose or a method for suppressing transplant rejection.

Moreover, the invention can suppress the transplant rejection using the transplant rejection suppressor and also can provide foods, drinks (health foods and the like), nutriments and the like useful for suppressing the transplant rejection.

Especially, the invention can effectively inhibit (suppress) the rejection of the transplantation for the purpose of treatment, improvement and/or prevention of diseases accompanied by rejection in transplantation of human organs such as human liver, heart, lung, kidney, skin, cornea and corneal epithelium and stem cell transplantation of tissues, especially the organ transplantation.

## Description

### Technical Field

The present invention relates to a novel method for controlling a function of macrophages (hereinafter sometimes referred to as "Mφ") , monocytes and dendric cells. More specifically, the invention relates to an orally-ingestible transplant rejection suppressor for suppressing acute or chronic rejection accompanied by human organ transplantation, pharmaceuticals (oral drugs, eye drops, infusions and the like) using the same, and foods, drinks, nutriments and the like having its activity. Further, the invention relates to a method for suppressing transplant rejection and a use of the active ingredient for the transplant rejection suppressor.

### Background Art

An immune system is a system for protecting the self from biological invasion of cells (cancer cells and the like) grown by infection with viruses, bacteria and the like from outside or by abnormal autogenous cells. However, this immune system becomes abnormal to act excessively or functions to remove a self ingredient, and further an exclusion function sometimes becomes rather deficient. Diseases that cause such state are generally called immune diseases. Examples thereof include a large number of diseases, for example, acute and chronic inflammatory diseases such as atopic dermatitis, hay fever, asthma and sarcoidosis, autoimmune diseases such as allergic diseases, chronic rheumatoid arthritis, diabetes (IDDM), SLE and chronic fatigue syndromes, hepatitis, hepatic cirrhosis, inflammatory bowel diseases (IBD) such as ulcerative colitis and Crohn disease, and cancer cachexia. These immune diseases have various causes. From triggering of inflammations accompanied by growth, differentiation and necrosis of specific cells through local production of cytokines or inflammatory mediators, systemic immunodefficiency and functional deficiency are induced. Meanwhile, in the transplant reaction of various organs such as kidney, liver, heart, lung, skin and cornea, it is well known that the graft is rejected or dropped by biological immune responses.

As immunocompetent cells, T lymphocytes and B lymphocytes are well known, and they exhibit various functions as cells responsible for cellular immunity and humoral immunity. Meanwhile, macrophages and monocytes (including dendric cells and Langerhans cells) are cells that deeply participate in cellular immunity and humoral immunity, and they are deeply related with immune diseases such as allergy and rheumatoid arthritis, cancer, bacteria infection and exclusion of non-self foreign bodies such as grafted tissues. The function of macrophages and monocytes is roughly classified into four types, secretory function, immunomodulating function centering on antigen presentation, function of treating and englobing foreign bodies and waste matters, and function of treating disorders of target cells. They are known to produce various molecules defining inflammation stage, for example, cytokines such as TNF, IL-12, IL-1, IL-6, TGFβ, IL-8 and IL-18, neopterine (NPT), hormone-like molecules such as dihydroxyepiandrostene (DHEA), arachidonic acid metabolites such as PGE2 and LTB4, complement molecules such as C5a and C3, active oxygen and active nitrogen. It is unknown whether these various functions are provided by a single type of macrophage/monocyte or by combination of macrophage (s) and/or monocyte(s) different in function. While lymphocytes are classified into distinctive subsets according to cell surface markers thereof and correspondence to functions thereof has been clarified, correspondence of various functions of macrophages or monocytes to cell subsets is completely unknown. Although macrophages and monocytes have quite an important role in triggering the foregoing inflammatory, allergic and immune diseases or progression of diseases of rejection after organ transplantation, the treatment, improvement and prevention of human diseases according to the supposed functional classification of macrophage and monocyte subsets, and the application to the transplantation field in particular have not been performed at all, and even the assumption has not been made.

In recent years, the deviation of the type of the helper T cell sub-group in the peripheral blood is being related with diseases. The helper T lymphocyte, a sub-group of T lymphocytes is further classified into two subsets, Th1 and Th2, and the presence ratio of these two types is proving to be an important index of an immunological function of organisms. Attempts are being made to establish a more appropriate therapy by diagnosis of conditions of diseases based on this index or by improvement in the presence ratio thereof. That is, it has been clarified that when a concentration of Th2 inducing IgE production from B cells is higher than that of Th1 (Th1 < Th2), allergic diseases are worsened. Attempts are being made to detect the immunological state by measuring Th1/Th2 or to suppress allergy by changing the ratio to Th1>Th2. On the contrary, diseases and the presence of a bioreaction caused by a dominant condition of Th1, including chronic rheumatoid arthritis and transplant rejection, are being indicated successively.

### Objects of the Invention

Although a balance of Th1 and Th2 is measured using biological materials to regulate functions of these two subsets with T lymphocytes as a target, this approach is now not successfully utilized in detection or diagnosis of local chronic inflammations or allergic diseases. The term, a Th1 type disease or a Th2 type disease has been lately used. However, these two types of diseases cannot necessarily be discriminated sharply.

The Th1/Th2 presence ratio is only an index of lymphocyte subsets, and the in-vivo dynamism of the lymphocyte subsets is actually related intricately with functions of cell groups such as dendric cells and Langerhans cells called accessory cells including macrophages and monocytes handled in the invention. Accordingly, it is difficult to properly diagnose the conditions of diseases only with the Th1/Th2 presence ratio and to conduct therapy on the basis of its information. As will be later described, the Th1/Th2 balance is controlled by the state of the function of macrophages or monocytes. Even though the balance is inclined to Th1>Th2 for therapy, the effect can hardly be provided in an intricate cytokine network by only this inclination. Thus, a new index for diagnosis and therapy has been in demand.

In macrophages deeply participating in the inflammation and rejection reaction, it has been clarified that the functions of cells are changed owing to environmental factors such as oxidative stress, cytokine stimulation and infection of viruses or bacteria. However, the correspondence of the functions to the classification of cell subsets of macrophages is totally unknown. In these functions and classifications, new findings are required, and obtainment of these findings leads to the development of an extremely useful new therapy.

### Disclosure of the Invention

The present inventors have assiduously conducted investigations to solve the foregoing problems, and have consequently obtained the following various findings. That is, they have tried to discriminate macrophages, monocytes and dendric cells having a strong activity of damaging tissues from macrophages, monocytes and dendric cells participating in tissue repair through a difference in redox state of macrophages, monocytes and dendric cells, and have enabled the same. A reduced glutathione content in macrophages, monocytes and dendric cells is employed as its index.

Glutathione is a tripeptide which is present in all mammalian cells, well known as an intrinsic antioxidant and has various functions in cells, such as removal of radicals or peroxides, metabolism of eicosanoid, for example, prostaglandin, detoxication of biological foreign bodies and amino acid transport. There are a reduced type (GSH) and an oxidized type (GSSG), and conjugated cycle is formed therebetween. In ordinary cells, the GSH concentration is overwhelmingly high in the reduced state, and it acts defensively against oxidative stress, especially H₂O₂.

Rude et al. have already reported that with respect to macrophages differentiated in the presence of GM-CSF and macrophages differentiated in the presence of M-CSF, the intracellular GSH concentration of the former is higher than that of the latter, and there is therefore a possibility that the difference in the intracellular GSH concentration might be related with the function of macrophages (Germann, T., Mattner. F., Partenheimer, A., et al.: Different accessory function for TH1 cells of bone marrow derived macrophages cultured in granulocyte macrophage colony stimulating factor or macrophage colony stimulating factor. Int. Immunol., 4:755, 1992; Frosch, S., Bonifas, U., Eck, H. P., et al. : The efficient bovine insulin presentation capacity of bone marrow-derived macrophages activated by grnulocyte-macrophage colony-stimulating factor correlates with a high level of intracellular reducing thiols. Eur. J. Immunol., 23 : 430, 1993). The present inventors have measured the reduced GSH content in macrophages and monocytes, and have found that in macrophages, a difference in GSH content leads to a significant difference in effect exerting an immunological function, that the immunological activity of organisms can be detected by this measuring method and the redox state can be artificially regulated with an orally-administrable specific low-molecular weight compound, that this method can be used in therapy of suppressing acute and chronic rejections accompanied by the organ transplantation, and that the substance (the foregoing specific low-molecular weight compound) used for this purpose can widely be applied as specific drugs, for example, pharmaceuticals and used by being contained in foods, drinks and nutriments showing such an activity.

On the basis of the foregoing findings, the present inventors have further conducted investigations, and have consequently found that by detecting the contents of oxidized glutathione and reduced glutathione in macrophage cells playing an important part in the inflammatory reaction, the non-uniform macrophage group can be classified into two types, namely, an oxidative macrophage and a reductive macrophage and the reductive macrophage induces local tissue inflammation accompanied by immune diseases or organ rejection reaction, that a Th1/Th2 balance participating in a balance of humoral immunity and cellular immunity is controlled by a redox state of macrophages, and that the redox state of macrophages plays an important part in diseases of the organ rejection reaction and the detection of the redox state and the artificial control and modification of this state are useful in diagnosis and therapy of the diseases, and that this control can easily be performed with the orally-ingestible low-molecular weight compound as noted above.

It is now considered that the Th1/Th2 balance is defined by the ratio how IL-6, IL-10 or IL-4 and IL-12 are produced in vivo. It has been already known that Th2 participating in humoral immunity is induced by the former three and Th1 participating in cellular immunity by IL-12, respectively. It has been clarified that IL-6, IL-10 and IL-12 are produced from macrophages. However, assuming the one subset of (the same) macrophage cells produce all of IL-6, IL-10 and IL-12, there should be one subset of macrophages that participate in both of the Th1 induction and the Th2 induction, which gives rise to a great contradiction in considering the biological immune response. The present inventors have found that IL-12 is produced by only the reductive macrophages having the high GSH content to cause the Th1 deviation and the production of IL-6 and IL-10 proceeds with the oxidative macrophages to induce the Th2 deviation. It has further been found that even in the presence of IFN γ which is a typical Th1 cytokine, when macrophages are inclined to an oxidized type, large amount of IL-6 inducing Th2 is produced by the action of IFNΥ.

On the contrary, it has been also found that when reductive macrophages are present, the character of reductive macrophages is all the more enhanced by IFNΥ, a typical Th1 cytokine. When IL-4, a typical Th2 cytokine acts on oxidative macrophages induced, the character of oxidative macrophages is all the more enhanced. These findings show that opposite immune responses of the humoral immunity and the cellular immunity are unequivocally defined by the redox state of macrophages, and these are important findings related with the basis of immunology. By applying the findings to suppression of the organ transplant rejection, a useful, original invention that replaces an ordinary chaotic antigen-non-specific immunosuppression method has come to be completed regarding a clinically quite important therapy, namely rejection suppression.

The invention has been thus completed on the basis of the foregoing many findings.

That is, the invention is as follows.

The invention resides in a transplant rejection suppressor, especially preferably an organ transplant rejection suppressor, characterized in that a substance having a function of decreasing a reduced glutathione content in cells which types are macrophages, monocytes or dendric cells is contained as an active ingredient.

With respect to the substance having a function of decreasing a reduced glutathione content, it is possible to use one or more of compounds that lower or decrease the reduced glutathione content in cells which types are macrophages, monocytes or dendric cells by at least 30% or so when incubated in vitro with macrophages for from 1 to 24 hours.

Preferable examples of the substance having a function of decreasing a reduced glutathione content in cells which types are macrophages, monocytes or dendric cells can include low-molecular weight compounds, for example, cystine derivatives such as N, N'-diacylcystine (diacetyl compound and the like) and esters thereof (such as monoethyl ester, dimethyl ester and diisopropyl ester), busulfan (BCNU), L-S, R-buthionine sulfoximine (BSO), derivatives thereof, and maleic acid diesters (dimethyl ester, diethyl ester and the like). These low-molecular compound can be used as the active ingredient either singly or as an admixture of two or more thereof.

Typical examples of the organ to be transplanted in the invention include liver, lung, kidney, liver, heart, skin, cornea and corneal epithelium. In the volume of the organ to be transplanted, the transplantation of the overall organ and the transplantation of a part thereof are included.

Further, the invention can also be applied to the rejection of the transplantation of stem cells in these tissues.

Typical examples of the rejection can include rejection to a minor antigen and rejection mainly including this rejection.

Especially preferable examples of the organ to be transplanted can include cornea, corneal epithelium and the like. The active ingredient of the invention can be used for the rejection to a minor antigen, preferably.

The transplant rejection suppressor of the invention can be used in the form of pharmaceuticals. In this case, it can be used in the form of any of oral drugs and parenteral drugs (eye drops, infusions (transfusion)and the like).

Eye drops are especially effective for suppressing the rejection in the corneal transplantation.

The abovementioned transplant rejection suppressor of the invention can also be used by being contained in foods, drinks or nutriments.

With respect to a more specific form, the invention can provide a transplant rejection suppressor usable in pharmaceuticals (including infusions) useful for therapy of suppressing the human organ transplant rejection or foods, drinks, nutriments and the like useful for suppressing the rejection by detecting the oxidized and/or reduced glutathione content in macrophage cells using body fluid/cell samples separated and collected from human beings to classify macrophages into oxidative macrophages and reductive macrophages having different functions (refer to Japanese Patent Kokai Publication JP-A-11-246435) and artificially deviating the presence ratio thereof to oxidative macrophages with the foregoing low-molecular compound or artificially removing reductive macrophages. The body fluid/cell samples separated and collected from human beings are cells separated from, for example, a peripheral blood, an abdominal cavity, a thoracic cavity, local tissues, a cavitas articulare and various organs.

It is described below that the transplant rejection suppressor of the invention in suppressing the organ transplant rejection is quite an excellent drug that has not existed so far.

Especially in the organ transplantation, unlike the foregoing various immune diseases, the first exposure to the donor antigen occurs in the transplantation operation, and the immunological treatment can easily be performed. When the organ transplantation is performed, an immunosuppressor is used for suppressing the rejection in the actual clinical treatment. This treatment involves problems of side effects such as antigen-non-specific suppression of all immune systems and susceptibility to infection. Accordingly, it is important to establish a method for selectively suppressing an immunoreaction only to a donor antigen, and this is the most ideal therapy. The present inventors have assiduously conducted investigations on the development of a therapeutic method for inducing immunological tolerance to a donor antigen in which the same constant take as in the autotransplantation is obtained without the need of an immunosuppressor, and have here completed the invention. It is believed that the development of this method is worthy to be called as the therapy of the 21st century. The merits of the therapy using the invention are that the reaction to the antigen of the transplantation organ is selectively inhibited (antigen specificity), and that after this cell appears, the more the exposure to the antigen, the stronger the inhibition mechanism (induction of Th2 cells to the donor antigen), and it can be applied to not only the transplantation of organs such as the kidney, lung, heart and liver but also the transplantation of cornea in high-risk eyes in which the antigen is presented quickly and the rejection occurs very easily.

The present inventors have developed a new method for suppressing rejection, and have completed a therapeutic method in which the deviation to the T helper 2 type (Th2) reaction is caused by artificially controlling the redox state of macrophages to suppress antigen-specific organ transplant rejection. They have succeeded till now in inducing antigen-specific Th2CD4+T cells or efficiently suppressing the rejection in a mouse corneal transplantation model and a mouse skin transplantation model. In this method, at the same time the organ transplantation is performed, the Th2 deviation reaction is locally induced to shift the reaction to the donor transplant antigen from Th1 to Th2 type. Since the rejection of the organ transplantation is mainly caused by a delayed type hypersensitive reaction that Th1 type CD4+T cells induce, the rejection can be selectively suppressed by inducing Th2 type T cells there against.

Regarding the donor antigen-specific rejection suppression by the Th2 deviation till now, administration of IL-4 or IL-10 in large quantities or gene introduction has been often attempted, but it does not influence a reaction in a local lymphonodus where is an actual antigen-presenting site. This is because a cytokine itself does not have an endocrine function in vivo, and mainly has a paracrine or autocrine function in the vicinity of cells. Besides, long-term continuous administration requires high cost and much labor. The invention is a method having a recipient with a transplanted organ to release Th2 cytokine in a local lymphonodus, and then a systemic side effect is decreased. Further, since a reaction to a donor is deviated to a reaction of suppressing rejection, suppression that always exceeds the rejection occurs. At present, it is only the present inventors who have established this method in the organ transplantation. The maximum merit of this method is that it can be performed using the reaction present in vivo without destroying the in-vivo immunological balance and therefore realized easily and safely at low costs. This is also the characteristic feature.

Especially, the corneal transplantation is safe transplantation having a high graft survival rate in comparison to other organ transplantations. The surgical operation is performed yearly in 50,000 cases in the United States and 2, 000 cases in Japan. Further, in recent years, the site in which corneal epithelium stem cells are present has been found, and a new technique has been established from the standpoint of cell biology. Thus, it has been possible to perform surgical treatment in diseases to which the operation was not applied in the past. Under these circumstances, the greatest problem at present is rejection to a graft. With respect to a clinical measure thereagainst, only local or systemic administration of steroid or cyclosporin in large quantities has been conducted, and their side effects have now been at issue.

Moreover, to enlarge applicable diseases, establishment of a transplant technique into high-risk eyes is deemed to be a great problem. In the transplantation into a cornea in which new blood vessels are invaded, antigen sensitization is quickly performed, and effector cells easily reach a donor cornea. In the transplantation into a cornea in which Langerhans cells float after inflammation, antigen sensitization occurs at an early stage.

With respect to problems in enlarging the technique, there are limbus transplantation and epithelium transplantation. In the limbus transplantation, Langerhans cells of a donor are incorporated, which makes it easy to present an antigen. Moreover, in both of the limbus transplantation and the epithelium transplantation, the transplantation site is the limbus rich in blood vessel tissue, so that it tends to be rejected. Besides, the corneal epithelium has a high antigenicity. In the antigen-non-specific immunosuppression method used so far, susceptibility to infection, diabetes, digestive ulcer, nervous diseases, obesity and renal insufficiency are pointed out as problems of the systemic side effect, and steroidal glaucoma, steroidal cataract and susceptibility to infection as problems of the local side effect respectively. These problems are also solved by practicing the invention.

High-risk eyes in which new blood vessels are present in a recipient's cornea tend to cause transplant rejection, and steroid or an immunosuppressor is used. However, there are still many problems. In the report on the corneal transplant rejection, it is known that compatibility of MHC alone (major histocompatibility complex) is not effective for reducing the rejection and compatibility of minor antigens except MHC significantly decreases the rejection. This is because a cornea has a special immunological atmosphere called an immune privilege site. That (1) the expression of MHC is low in the corneal tissue, (2) blood vessels or lymph vessels are absent and (3) antigen-presenting cells are absent in the corneal tissue, are concerned. Because of these special environments, the donor antigen in the corneal transplantation is considered to be presented to T cells of a recipient mainly by antigen-presenting cells of the recipient (Indirect pathway of allorecognition). In this system, the recipient is sensitized, and CD4+T cells that undergo the antigen presentation cause a delayed type hypersensitive reaction (DTH) to induce rejection. In the corneal transplantation, it is considered that these procedures are conducted more easily because the cornea is an immune privilege site.

The corneal transplantation is the safe transplantation having the best graft survival rate among organ transplantations and has been often performed in the world so far. In recent years, however, the concept of the transplantation has been developed, and diseases to which the transplantation technique is applied or the transplantation method has been enlarged. Accordingly, the corneal transplantation in which the rejection tends to occur has been increased.

The significance of the invention is described by taking the corneal transplantation as an example. By combining the induction of the strong Th2 reaction with the antigen re-exposure in an appropriate stage, (1) only the reaction to a corneal transplantation antigen can be inhibited (antigen specificity), (2) since cells reactive with Th2 are induced to a donor antigen, it is possible to cause the inhibition that always exceeds the rejection, and (3) the invention can also be applied to high-risk eyes in which an antigen is quickly presented and the rejection occurs at a high rate.

In the corneal transplantation, it should be considered that a functional role of an organ is maintained. In the corneal transplantation, the shape and the function of the graft are simple, and antigen-presenting cells of a donor are absent. Accordingly, it is the most suitable transplantation as a model of rejection to a minor antigen. Therefore, the method for suppressing the rejection which is established in the corneal transplantation can also be applied well to the other organ transplantations, and it is expected to give an epochal turning point also to the suppression of the rejection in organ transplantations in future. The excellent effects obtained in the invention, especially novel points on the suppression of the corneal transplant rejection and the fixing of the graft as typical examples thereof are: (1) a hitherto-totally-unknown vital effect in a therapeutic method that an immunoreaction is changed by treating antigen-presenting cells, (2) a breakthrough of the point in which there was only antigen-non-specific immunosuppression in an ordinary method and (3) a breakthrough of the point in which induction of tolerance by an antibody or the like was used at research levels but was not put to practical use. Further, the better points are: (1) there is a possibility of performing antigen-specific immunosuppression from an ordinary nonspecific immunosuppression method and (2) the use of an immunosuppressor can be limited. Practically, advantageous points are: (1) antigen-specific suppression, (2) low costs achievement, (3) after the treatment according to the invention, the more the exposure to an antigen, the stronger the suppression mechanism (induction of Th2 cells to a donor antigen), and (4) applicability to high-risk eyes in which an antigen is quickly presented and rejection tends to occur.

With respect to a method for measuring the glutathione, the oxidized or reduced glutathione content is directly measured biochemically by an enzyme recycling method (Active Oxygen Experiment Protocol (Saibo Kogaku, separate volume), Shujun Sha, pp. 84-88, 1994, ANALYTICAL CHEMISTRY, vol. 106, pp. 207-212, 1980, and CELLULAR IMMUNOLOGY, vol. 164, pp. 73-80, 1995). In addition, indirect measurement, for example, measurement using a monoclonal antibody or polyclonal antibody specific to oxidative or reductive macrophages, or measurement using a reagent that is specifically reacted with GSH to form a complex and emit fluorescent light by laser beam excitation, such as monochlorobimane, is also available.

According to another embodiment, the invention resides in a method for suppressing transplant rejection, characterized by ingesting or administering to a living body the substance having a function of decreasing a reduced glutathione content in cells which types are macrophages, monocytes or dendric cells.

In the ingestion or administration form, the transplant rejection suppressor can be employed. Especially, it can preferably be ingested or administered in the form of pharmaceuticals or in the form used in foods, drinks and nutriments.

According to still another embodiment, the invention resides in a use of the substance (active ingredient) having a function of decreasing a reduced glutathione content in cells which types are macrophages, monocytes or dendric cells for a transplant rejection suppressor or the production thereof.

The transplant rejection suppressor is as described earlier, and the form of pharmaceuticals or the form used in any of foods, drinks and nutriments, as noted above, can be mentioned as a preferable example.

### Embodiments of the Invention

The embodiments of the invention are described.

Glutathione in the invention has another name, 5-L-glutamyl-L-cysteinylglycine. It is an SH compound present in vivo at the highest concentration, and generally referred to as GSH. Glutathione is classified into reduced glutathione and oxidized glutathione depending on the oxidized state of its molecule. Reduced glutathione is the foregoing glutathione (GSH), and oxidized glutathione is called as glutathione disulfide as another name, and referred to as GSSG.

A method for classifying macrophages in the invention is a method based on the foregoing detection of the contents of reduced glutathione and oxidized glutathione. It is known that macrophages liberate or release signal transmitters such as various cytokines and inflammatory mediators from the cells, but it was found in the invention for the first time that depending on the activated or differentiated state of macrophages, the release of such transmitters, the released amount and the function vary. The classification method in the invention has classified macrophages into oxidative macrophages and reductive macrophages upon focusing on the contents of oxidized glutathione and reduced glutathione in macrophage cells.

In reductive macrophages, the reduced glutathione content in cells is relatively larger than that in oxidative macrophages, whereas in oxidative macrophages, the reduced glutathione content is relatively smaller than that in reductive macrophages. Further, owing to the difference in the reduced GSH content, there is a difference in activation of transcription control factors between reductive macrophages and oxidative macrophages which leads to a difference in gene expression of cytokines or inflammatory transmission factors, a change in type or amount of inflammatory cytokines or inflammatory mediators produced and a change in inflammation nature.

In oxidative macrophages, inflammatory cytokines and mediators such as IL-6, IL-1, IL-8, IL-10, TNF, hydrogen peroxide, superoxide, PGE2 and PDGF are produced, whereas in reductive macrophages, nitrogen monoxide (NO), IL-12, IL-18, LTB4 and the like are produced, inducing tissue disorders. Further, oxidative macrophages and reductive macrophages are converted by stimulation or the like. For example, reductive macrophages can be converted to oxidative macrophages by being artificially stimulated with LPS (lipopolysaccharide) inducing inflammation or septic shock, PMA (phorbol ester) or cytokines such as IL-4 and TGFβ. On the contrary, oxidative macrophages can be converted to reductive macrophages by adding IFNΥ, IL-2, letinan (LNT) as an antitumor polysaccharide or an antioxidant such as lipoic acid.

According to the invention, it is advisable that after measuring the reduced glutathione content in macrophages and monocytes by, for example, the foregoing method, a substance which has a function of decreasing the reduced glutathione content in macrophages and monocytes, especially a low-molecular weight substance which maintains the activity even through oral administration and is preferably formulated into pharmaceuticals in a usual manner, is administered to patients every day or at appropriate intervals while monitoring the condition of the disease. In a chronic stage, the effect becomes remarkable in the long-term continuous administration.

In the invention, the substance having the function of decreasing the reduced glutathione content in macrophages, monocytes or dendric cells can preferably be selected from among low-molecular weight compounds such as N, N'-diacylcystine, its esters, BCNU, BSO and maleic acid diesters. However, these are not critical. All compounds having the function of decreasing the reduced glutathione content in cells by in-vitro incubation along with macrophages for some hours (1 to 24 hours or so) are included in the active ingredient used in the invention. When these active ingredients are used in the invention, they can be used either singly or in combination (mixture). The effect thereof can be judged by collecting monocytes from an inflammatory local portion or a peripheral blood after ingestion or administration and detecting the change in reduced glutathione content in cells in comparison to the content before the treatment. The usefulness as the transplant rejection suppressor is thereby clearly judged, and it is effective against the intended diseases in the invention.

In regard to the intended diseases available, the invention can widely be applied to diseases accompanied by rejection caused because of the deviation of the Th1/Th2 balance to Th1, such as rejections in heart, lung, kidney, liver and skin transplantations, corneal transplantation in which compatibility of a minor antigen becomes a problem, corneal epithelium transplantation and corneal stem cell transplantation.

The transplant rejection suppressor handled in the invention can singly be administered in the actual medical site. The orally-ingestible transplant rejection suppressors included in the invention can be used in combination, or the transplant rejection suppressor can be mixed with, or used in combination with, other substances which are not orally ingestible but decrease the reduced glutathione content in any cells of macrophages, monocytes and dendric cells by a different functional mechanism, for example, in-vitro and in-vivo substances, such as steroidal compounds and cytokines typified by IL-4, IL-10 and TGF-β. It has been also found during the process of the invention that these cytokines themselves decrease the reduced glutathione content in macrophages, monocytes and dendric cells, and it backs up the usefulness and the scope of the invention. Further, the transplant rejection suppressor handled in the invention can also be used as drops either singly or in combination with drugs employed in the ophthalmic region.

When there is a difference in the reduced glutathione content in cells, that is, when macrophages, monocytes and dendric cells having the high reduced GSH content (reductive macrophages) and macrophages, monocytes and dendric cells having the low reduced GSH content (oxidative macrophages) are present, the selective removal of macrophages, monocytes or dendric cells (reductive macrophages) having the high reduced GSH content is also included in the invention. A substance used at this time may be either a low-molecular weight compound or a high-molecular weight compound, and antibodies and derivatives thereof are especially efficient.

As already stated, the correspondence between the various functions of macrophages and monocytes (including dendric cells) and the cell subsets has been to date completely unknown. Accordingly, although the antigen-presenting function and the accessory function of macrophages, monocytes and dendric cells play quite an important part in the progression of diseases in the transplant rejection, the application of the functional classification to the suppression of the transplant rejection by assuming the presence of macrophage and monocyte subsets has not been attempted at all, and even the assumption has not been made. In the invention, it has been found that the non-uniform macrophage group has been classified into two types, namely oxidative macrophages and reductive macrophages by detecting the contents of oxidized glutathione and reduced glutathione in macrophages, monocytes and dendric cells that play an important part in, for example, the organ rejection and reductive macrophages participate in Th1 deviation related with cellular immunity and that the redox state of macrophages plays an important part in diseases of the organ transplant rejection. In order to artificially control the presence ratio of these two types of macrophages, the selective removal of one of two types of macrophages is also quite an effective method in addition to the use of the foregoing low-molecular weight compounds as pharmaceuticals. It is easily conceivable to those skilled in the art that one of two types of macrophages or an antibody to a marker expressed in large quantities may be used.

Since animal model experiments of organ transplantation have been widely conducted, the essence of embodiments of the invention is described with respect to the corneal transplantation and the skin transplantation.

In estimating the induction of the cornea take in the corneal allograft, mice, 8 to 10 weeks old, are used, a BALB/c (H-2d) mouse is used as a recipient, and a B10.D2 (H-2d) mouse only different in minor antigen is used as a donor.

The corneal transplantation inducing corneal new blood vessel of a high-risk eye that tends to cause rejection at a high rate in an early stage in a recipient: a corneal parenchyma is sutured with three stitches using a 11-0 nylon yarn which is allowed to stand for 14 days and the nylon yarn is removed in the corneal transplantation, is performed as follows. To a recipient mouse are intraperitoneally administered 3 mg of ketamine and 0.0075 mg of xylazine for anesthesia, and transplantation is performed. A central portion of cornea in a donor mouse is cut out to a circle having a diameter of 2 mm to form a graft. A central portion of cornea in a host mouse is cut out to a diameter of 2 mm. The donor cornea is sutured on margins with eight stitches using a 11-0 nylon yarn. After the surgical operation, an antibiotic ointment is coated thereon, and the eyelids are sutured with a 8-0 nylon yarn. The eyelid suture is removed after 72 hours from the surgical operation, and the corneal suture is removed on day 7 after the transplantation.

In the judgement of the corneal rejection, monitoring is performed twice a week using a slit-lamp microscope. The rejection is judged by scoring a corneal opacity with six grades. In connection with a time capable of judgement, the score of 3 or more on or after day 14 from the transplantation and the score of 2 or more on or after day 21 are judged to indicate the rejection. (0: Transparent take, 1: Superficial cornea is slightly opaque and iris blood vessel and iris leopard can be clearly seen through, 2: Stroma is slightly opaque and iris blood vessel and iris leopard can be seen through, 3: Corneal parenchyma is medially opaque and iris blood vessel and iris leopard cannot be seen through, 4 : Corneal parenchyma is highly opaque and iris margin cannot be seen through, and 5: Cornea is completely opaque and anterior chamber cannot be seen through.)

A method for administrating a drug (active ingredient used in the invention) is understood from, for example, the following method.

In a method of one administration, a drug is dissolved or suspended in a physiological saline solution to a predetermined concentration, and 0.5 ml thereof is then intraperitoneally injected in a mouse. The injection is conducted three times, 7 days, 4 days and 1 hour before the corneal transplantation. 0.5 ml of a physiological saline solution is intraperitoneally injected in a control mouse. A significant difference in graft survival rate of the corneal transplantation was comparatively detected by the Breslow-Gehan-Wilcoxon test using Kaplan-Meier survival curves. A P value of less than 0.05 is defined as significant.

The significances of the corneal epithelium transplantation are as follows.

The cornea consists of five layers: corneal epithelium, Bowman membrane, Stroma, Descemet membrane and corneal endothelium as viewed from the outside. The corneal epithelium has a high growth capacity, and cells are supplied from stem cells present in the limbus. Accordingly, the corneal epithelium replaces a host corneal epithelium within a few months by using even a penetrating keratoplasty or lamellar keratoplasty which is transplantation of a center part of cornea. Further, in the five-layer structure, the corneal epithelium has the highest antigenicity, so it tends to be a target of early rejection after the transplantation. Finally, the corneal epithelium is a layer which is quite easily dropped due to inflammation or rejection; therefore, it is very difficult to maintain a donor epithelium. At present, a corneal transplantation technique has been used for replacement with a normal tissue or for supplementation of a deleted tissue. The significances of performing the corneal transplantation are that (1) sight is improved by making clear an opaque cornea (treatment mainly by penetrating keratoplasty or lamellar keratoplasty), (2) on a cornea in which endothelial cells, which could not proliferate, are reduced by surgical invasion or diseases causing bullous keratopathy, sight improvement or pain release is performed for supplementing the endothelial cells (treatment mainly by penetrating corneal transplantation), and (3) on a cornea in which an epithelium including stem cells is damaged, invasion of epithelial cells of conjunctiva is inhibited, and it is coated with transparent epithelial cells of cornea. The corneal epithelium transplantation is mainly used for the purpose (3). However, there are many cases with the corneal opacity, and this transplantation has often the significance (1).

As a surgical treatment of eye surface diseases in which a corneal epithelium is deleted and the deleted portion is covered with a conjunctival epithelium to cause opacity, a lamellar keratoplasty is performed. The lamellar keratoplasty is a method in which only an epithelium of limbus and a superficial stroma are cut out from a donor cornea and transplanted into a host limbus, and its purpose is to keep proliferating the graft corneal epithelium and to maintain the host corneal epithelium. However, in intractable keratoconjunctival diseases having intense inflammation, such as Stevens-Johnson syndromes, optic pemphigoid and corneal chemical injury, the prognosis thereof is quite bad. The biggest reason is that an allo-corneal epithelium having high antigenicity is recognized by host immune system to undergo rejection. Further, since the transplantation site is a limbus where blood vessel tissue and Langerhans cell is rich, antigen sensitization or rejection to a donor antigen tends to occur. Moreover, a complication caused by administering large amount of immunosuppressor systemically or locally after the surgery for preventing rejection is also a great factor for the bad prognosis. Examples of a systemic steroidal side effect include susceptibility to infection, diabetes, digestive ulcer, nervous diseases and obesity, and examples of a local eye side effect include steroidal glaucoma, steroidal cataract and susceptibility to infection. Accordingly, the method of the invention in which only the immunoreaction to the donor antigen is selectively inhibited is considered to be ideal. It means that a new safe therapeutic method concerning intractable keratoconjunctival diseases which have not been positively treated at present can be provided, and the social contribution thereof is considered to be quite high. The lamellar keratoplasty is performed as follows. A bulb of eye of a donor mouse is cut out in the limbus using Vanas scissors to form a full thickness corneal graft. The graft is further divided into corneal grafts having a size of 1.0 mm x 2.5 mm. The divided donor corneal grafts are temporarily stored in RPMI medium until the transplantation operation. A host mouse is put under anesthesia, and the corneal epithelium is carefully separated from the center of cornea to the limbus using sclera scissors. A half layer of the sclera where the limbus is shifted to the conjunctiva is cut out to a width of approximately 1.0 to 1.5 mm. The three corneal grafts of the donor are sutured on the sclera cutout site using two 11-0 nylon yarns. Incidentally, regarding the host bulb of eye, only the right eye is used.

The corneal stem cell transplantation is described. In diseases such as Stevens-Johnson syndromes, optic pemphigoid and corneal chemical injury, the corneal epithelium including stem cells is dropped which causes invasion of an opaque conjunctival epithelium with blood vessels. In diseases in which a corneal epithelium is genetically abnormal, such as gelatinous drop-like corneal dystrophy, stem cells per se are abnormal. As a method for treating these diseases, there is only a method in which stem cells of a normal corneal epithelium in a limbus of a normal eye are transplanted to always supply normal corneal cells. However, the allo-corneal epithelium transplantation tends to be rejected, and it is hard to maintain the graft. Accordingly, the suppression of the rejection using the transplant rejection suppressor of the invention is expected to change the therapy of these diseases.

The model system of the skin transplantation for confirming the effects of the invention is as follows, and the outstanding effects thereof can easily be understood.

Mice, 8 to 10 weeks old, are used, a BALB/c (H-2d) mouse is used as a recipient in transplantation, and a B10.D2 (H-2d) mouse different in minor antigen only is used as a donor. To the recipient mouse, 3 mg of ketamine and 0.0075 mg of xylazine for anesthesia are intraperitoneally administered, and transplantation is performed. The skin on the body wall of the donor mouse is transplanted on the thoracic wall (8 x 8 mm) . A gauze having a larger size than the graft covers there, and an antibiotic ointment is applied. The graft is fastened with a cast to complete the operation. The cast is removed after 7 days, and the rejection is then observed every day.

The organ transplant rejection suppressor included in the invention can be applied to wide-ranging transplantations, which is clear from the point that secretion of inflammatory factors (transfer factors) from macrophages is basically controlled. For example, non-steroidal anti-inflammatory drugs (aspirin and the like) are said to exhibit their pharmaceutical effects by inhibiting prostaglandin production or liberation. Meanwhile, antioxidants such as vitamin E exhibit their pharmaceutical effects by inhibiting production of active oxygen. Thus, such function is only controlling a part of various functions of macrophages. Accordingly, the effects thereof are not sufficient; especially, in the chronic rejection, the drugs are scarcely effective against the chronic rejection. On the contrary, the organ transplant rejection suppressor included in the invention is based on the control of the redox state of macrophages, and it can inhibit many productions of harmful inflammatory factors at a time. It can be said to basically change the ordinary concept of drugs.

As stated above, the useful efficacy in the medical site of the transplant rejection suppressor of the invention, preferably the organ transplant rejection suppressor included in the invention is self-evident from its novel functional mechanism, and it is effective against both the acute and chronic rejections. Among others, to diseases related with the organ transplantations of the lung, heart, kidney, liver, skin, cornea and the like in which the rejection caused by the Th1 deviation mainly occurs, the transplant rejection suppressor of the invention can be widely applied.

It has been thus far described that the transplant rejection suppressor of the invention is quite effective and excellent when used as, for example, pharmaceuticals in the organ transplantation. Since the transplant rejection suppressor of the invention contains the orally-ingestible substance as an active ingredient, its use is not limited to pharmaceuticals (internal drugs, infusions, drops and the like) in the medical site. That is, nutriments, foods and drinks which contain substance(s) having a function of decreasing the reduced glutathione content in human macrophages and monocytes either singly or in combination and which have the transplant rejection suppressory effect can be also provided, for example, in the form of medical foods, health foods and food for specified health uses, and these are included in the invention too.

The products may be contained in a liquid ingredient or take the form of solid foods.

Intended diseases are the same as in providing pharmaceuticals.

The formulation in using as pharmaceuticals is briefly described.

Various materials for formulation (as adjuvant and the like) which are pharmacologically acceptable can be contained in addition to the active ingredients (one or plural) used in the invention. The materials for formulation can be properly selected according to the dosage form. Examples thereof can include excipients, diluents, additives, disintegrants, binders, coating agents, lubricants, smoothing agents, glossy agents, flavors, sweeteners, solubilizers and the like. Specific examples of the materials for the formulation can include magnesium carbonate, titanium dioxide, lactose, mannitol, other saccharides, talc, milk protein, gelatin, starch, cellulose, derivatives thereof, animal and vegetable oils, polyethylene glycol, and solvents such as sterile water, monohydric alcohols and polyhydric alcohols, for example, glycerol.

The transplant rejection suppressor of the invention can be prepared in various forms of pharmaceutical preparations which are known or will be developed in future, for example, in forms of oral administration (internal drugs), intraperitoneal administration, percutaneous administration, suction administration and eye drops. For formulating the transplant rejection suppressor of the invention into these various forms of pharmaceutical preparations, methods which are known or will be developed in future can properly be employed.

These various forms of pharmaceutical preparations can include appropriate solid or liquid preparation forms such as granules, powders, coated tablets, tablets, (micro) capsules, suppositories, syrups, juices, suspensions, emulsions, drops, injection solutions and drugs for delaying release of an active substance.

It is a matter of course that the transplant rejection suppressor of the invention in the above-listed preparation forms has to contain the active ingredients in a pharmaceutically effective dose.

The dose of the transplant rejection suppressor in the invention is properly selected according to the type of the active ingredient used, the type of the rejection, the degree of its state, the form of the preparation, the side effect, the extent thereof and the like. For example, in case of a preparation containing N, N'-diacetylcystine dimethyl ester as an active ingredient, it can be orally administered to a patient in a dose of, preferably from 1 mg to 10 g, more preferably from 3 mg to 1 g, further preferably from 9 to 270 mg per day in terms of a net weight of the active ingredient. Further, in case of the serious condition, the dose can be increased more. With respect to the administration time and period, it can be administered once per several days or once a day. Usually, it is administered several times a day, for example, twice to four times. In the intravenous administration, the dose may be one-tenth to one-twentieth the dose in the oral administration.

When other ingredients are used, necessary preparations can be formed on the basis of these matters, upon using a known formulation technique and according to various dosage forms.

When the transplant rejection suppressor of the invention is used by being incorporated into foods, drinks and nutriments, the dose is determined by referring to the dose per day in the oral administration as described above on the use as pharmaceuticals. The content of the active ingredient occupied in products (pharmaceuticals, foods, drinks and nutriments) can optionally be selected. It is preferably approximately from 0.1 to 50% by weight, more preferably approximately from 0.2 to 40% by weight, further preferably approximately from 1 to 5% by weight.

As stated above, the invention resides in, according to another embodiment, a method for suppressing transplant rejection, characterized by ingesting or administering in vivo a substance having a function of decreasing a reduced glutathione content in any cells of macrophages, monocytes and dendric cells, and according to still another embodiment, a use of a substance (active ingredient) having a function of decreasing a reduced glutathione content in any cells of macrophages, monocytes and dendric cells for a transplant rejection suppressor or the production thereof.

These inventions can readily be practiced on the basis of the foregoing description on the transplant rejection suppressor of the invention or Examples to be described later and by referring to ordinary known techniques, if required.

### Preferred Embodiments

The invention is illustrated more specifically below by referring to Examples. However, these do not limit the scope of the invention.

### (Example 1)

Detection of functions of oxidative macrophages and reductive macrophages

### (Method)

By adhering peritoneal exudate cells to the plastic surface, then reacting the cells with 10 µ_{M} of monochlorobimane at 37°C for 30 minutes and conducting analysis by ACAS, it was found that oxidative macrophages were induced by intraperitoneally administering 20 µg of LPS (lipopolysaccharide) to mice and reductive macrophages were induced by likewise intraperitoneally administering 100 µg of LNT three times every other day. The increase in amount of oxidative macrophages can easily be detected visually from the fact that almost no reaction product is observed, that is, a gray or blue image is obtained, and the increase in amount of reductive macrophages from the fact that a red or yellow image can be obtained, respectively.

Therefore, NO, IL-6 and PGE2 produced by inducing peritoneal exudate adhered cells into oxidized cells and reduced cells were measured as followings.

### (1) Material

Cells: peritoneal exudate adhered cells obtained by being stimulated in the foregoing manner, namely macrophages, were added to a 96-well microplate at a concentration of 1 x 10⁵ cells/200 µl.
Medium: phenol-red-free RPMI 1640 200 µl/well
LPS: lipopolysaccharide (made by Sigma)(origin: E. coli) 100 ng/ml
IFNY: 100 units/ml

### (2) Incubation method

Incubation in a 5% CO₂ incubator at 37°C for 48 hours.

### (3) Measuring method

After completion of the incubation, the culture supernatant was recovered. An amount of IL-6 produced was measured by cell growth assay using MH60, an IL-6-dependent cell strain, an amount of PGE2 produced using an ELIZA kit, and an amount of NO produced using a Griess-Romijn's reagent respectively by methods which are routinely performed by those skilled in the art.

### (Results)

It is clear that oxidative macrophages and reductive macrophages are different in productivity and type of inflammatory cytokine IL-6, inflammatory mediator PGE2 and NO produced. That is, in oxidative macrophages, the productivity of IL-6 as Th2 cytokine and the productivity of PGE2 which is immunosuppressive to inhibit Th1 induction are increased and the productivity of NO to act on tissue disorder is decreased. In contrast to this, in reductive macrophages, the productivity of NO is increased, and the productivity of PGE2 and the productivity of IL-6 are inhibited. There is clearly a functional difference between both macrophages.

### (Example 2)

Detection using gene knockout immunodeficient disease model animals

Analysis at a substance level to seek why there is a difference in productivity of an inflammatory mediator or cytokine between oxidative Mφ and reductive Mφ is important to clarify a rejection mechanism. Generally, stimulus (ligand or the like) from outside is transmitted into inside the cells via receptors on cell surfaces. Various kinases are activated by signals from receptors, and transcription factors are further activated. Transcription factors are shifted into nuclei and bound to target genes to express new characters. According to recent researches, it is being clarified that a redox system in cells participates in activation of transcription factors, shifting into nuclei and binding to genes (refer to ANNUAL REV. IMMUNOLOGY, vol. 8, pp. 453-475, 1990, EMBO J., 10, 2247-2251 (1991)). It is now unclear how the redox system in cells participates in the gene expression system via receptors in Mφ. As one method for clarifying the same, Mφ was prepared from a knockout mouse deficient in molecules participating in a signal transduction system from receptors, and the function of the redox system was analyzed. Specifically, common gamma (common Υ chain) (Υc) used as a receptor constituting molecule of IL-2, IL-4, IL-7, IL-9 and IL-15 in common and Jak3 as a molecule which presents in the downstream thereof, and transfers a signal from Υc were used as target molecules.

### (Cytokines and stimulator)

A recombinant mouse IFNΥ was made by Genzyme. Recombinant human IL-2 and human IL-6 were made by Ajinomoto Co., Inc. A recombinant human IL-12 was made by Pharmingen.

A product derived from E. coli. 055; B5, as produced by Difco, was used as LPS. A preparation produced by Ajinomoto Co., Inc. was used as LNT.

### (Mice used)

Υc knockout mice were obtained from Prof. Sugamura, Medical Department, Tohoku University, and Jak3 knockout mice were obtained from Prof. Saito, Medical Department, Chiba University.

As wild type mice used for mating and as control, C57BL/6 mice bought from CRJ were used.

### (Collection of peritoneal Mφ)

Collection of peritoneal cells was conducted by injecting 5 ml of ice-cooled, phenol-red-free DMEM medium (made by Nikken Seibutsu) into an abdominal cavity of a mouse sacrificed with ether using a syringe fitted with a 22-gauge needle, squeezing it and then extracting the medium.

### (Quantitative determination of IL-6)

A stimulator was added to 1 x 10⁶ Mφ, and the mixture was incubated in a CO₂ incubator at 37°C for 2 days. After centrifugation, the culture supernatant was collected.

The quantitative determination of IL-6 was conducted using mouse hybridoma MH60 cells grown dependently on IL-6 (refer to J. EUR. IMMUNOL., vol. 18, PP 951, 1988). 100 µl of the culture supernatant was added to 100 µl of the MH60 cell solution adjusted to a concentration of 1 x 10⁵ cells/ml with a 10% FCS-containing RPMI medium, and the mixture was incubated in a CO₂ incubator at 37°C for 2 days. Then, 10 µl of MTT (made by Sigma) adjusted to a concentration of 5 mg/ml with the same medium was added thereto, and the mixture was reacted at 37°C for 5 hours. After completion of the reaction, the reaction mixture was centrifuged to remove 160 µl of the supernatant. 100 µl of hydrochloric acid-propanol was added thereto, and it was suspended therein with a pipette man to dissolve cells. Immediately after the dissolution, absorbance of 570 nm was measured by an immunometer (manufactured by Bio-Rad).

### (Measurement of NO₂ concentration)

A stimulator was added to 1 x 10⁶ Mφ, and the mixture was incubated in a CO₂ incubator of 37°C for 2 days. After centrifugation, the culture supernatant was collected.

To 100 µl of the culture supernatant, 100 µl of a Griess-Romijn's reagent (made by Wako Pure Chemical Industry, Ltd.), adjusted to a concentration of 50 mg/ml with distilled water, was added, and the mixture was reacted at room temperature for 15 minutes. After completion of the reaction, an absorbance of 540 nm was measured. Incidentally, as a standard, NaNO₂ was used.

### (Detection of intracellular GSH by ACAS)

300 µl of a cell suspension having cell concentration of 3 x 10⁵ cells/ml was prepared in RPMI 1640 medium (phenol-red-free), and it was charged into a chambered coverglass (#136439 manufactured by Nunc) and incubated in a CO₂ incubator of 37°C for 2 hours. The culture solution was washed with the same medium, and 300 µl of 10 µM monochlorobimane (made by Molecular Plobe) prepared with the same medium was added thereto. The mixture was charged into a CO₂ incubator of 37°C, and reacted for 30 minutes. An intensity of fluorescence was then measured by ACAS. By the way, UV laser was used in ACAS.

### (Quantitative determination of IL-12)

The quantitative determination of IL-12 was conducted by bioassay using human T cell strain 2D6 cells (refer to J. LEUKOCYTE-BIOLOGY, vol. 61, PP 346, 1997).

The 2D6 cells cultured in RPMI 1640 medium containing 500 pg/ml of recombinant human IL-12, 50 µM 2-mercaptoethanol and 10% FCS (bovine fetal serum) were shifted into a tube and centrifugally washed three times with the same medium from which IL-12 was removed, and the cell concentration was adjusted to 1 x 10⁵/ml. The cell suspension was added in an amount of 100 µl to each well of a 96-well flat bottom plate in which 100 µl of a sample diluted in series with RPMI 1640 medium containing 50 µM 2-mercaptoethanol and 10% FCS was previously charged in each well. The mixture was then charged into a 5% CO₂ incubator at 37°C to conduct the incubation for 48 hours. In the last 6 hours, ³H-TdR was pulsed (a substance adjusted to 370 kBq/ml with RPMI 1640 medium containing 50 µM 2-mercaptoethanol and 10% FCS was added in an amount of 50 µl each). The cells were harvested, and a radioactivity was measured with a β counter (Matrix 96 : manufactured by Packard) .

### (Measurement of GSH concentration of Mφ prepared from knockout mice)

Peritoneal cells of each knockout mouse were prepared, and the GSH content in cells was analyzed by ACAS using an MCB reagent. In any of the mice in comparison to the control mice (C57BL/6), the reduced glutathione content was significantly decreased.

### (Function of Mφ prepared from knockout mice)

Peritoneal cells were prepared from wild type mice (C57BL/6) and the respective knockout mice, and stimulated with LPS, IL-2, IFNΥ and a combination thereof to measure the NO productivity, the IL-6 productivity and the IL-12 productivity. With respect to the NO productivity, almost no productivity was observed in any mice without stimulation. In the stimulation with LPS and IFNΥ in combination, almost no effect by the combined use of IL-2 was observed in Υc knockout mice, and the NO productivity was decreased to less than half in comparison to control mice. In Jak3 knockout mice as well, the same results as in Υc were found in Jak3 knockout mice. Then, the IL-6 productivity was analyzed. In the LPS stimulation, the productivity was increased in Υc knockout mice (962 pg/ml relative to 81 pg/ml in control mice) . In the IFNY stimulation, the productivity was increased in Υc knockout mice. The results were the same as observed in the inhibition pattern of the NO productivity. Next, the IL-12 productivity was examined in the stimulation with LPS and IFNΥ. However, no productivity was observed at all in any of the mice. This shows that in the sick animals of gene knockout mice used here, the amount of oxidative macrophages is increased, the humoral immunity mainly caused by Th2 is increased and the cellular immunity caused by Th1 is decreased. In the sick animal model as well, it is clearly shown that the invention is novel and significant in the pathological diagnosis of immune diseases.

### (Example 3)

Induction of take of a corneal graft by induction of oxidative macrophages

Mice, 8 to 10 weeks old, were used, a BALB/c (H-2d) mouse was used as a recipient of transplantation, and a B10.D2 (H-2d) mouse different in minor antigen only was used as a donor.

A corneal new blood vessel of a high-risk eye in which rejection tends to occur at a high rate in an early stage is induced into the recipient. In the induction of the corneal new blood vessel, a corneal parenchyma was sutured with three stitches using a 11-0 nylon yarn which was allowed to stand for 14 days. The nylon yarn was extracted in the corneal transplantation. 3 mg of ketamine and 0.0075 mg of xylazine for anesthesia were intraperitoneally administered to the mouse, and the transplantation was performed. A central portion of cornea in the donor mouse was cut out to a circle having a diameter of 2 mm to form a graft. A central portion of cornea in the recipient mouse was cut out to a diameter of 2 mm. The donor cornea was sutured on margins with eight stitches using a 11 - 0 nylon yarn. After the surgical operation, an antibiotic ointment was coated thereon, and eyelids were sutured with a 8-0 nylon yarn. The eyelid suture was removed after 72 hours from the surgical operation, and the corneal suture was removed on day 7 after the transplantation. In the judgement of corneal rejection, monitoring was performed twice a week using a slit-lamp microscope. The rejection is judged by scoring a corneal opacity with six grades. In connection with a time capable of judgement, the score of 3 or more on or after day 14 from the transplantation and the score of 2 or more on or after day 21 are judged to indicate the rejection. (0: Transparent take, 1: Superficial cornea is slightly opaque and iris blood vessel and iris leopard can clearly be seen through, 2: Corneal parenchyma is slightly opaque and iris blood vessel and iris leopard can be seen through, 3: Corneal parenchyma is medially opaque and iris blood vessel and iris leopard cannot be seen through, 4: Corneal parenchyma is highly opaque and iris margin cannot be seen through, and 5: Cornea is completely opaque and anterior chamber cannot be seen through.)
With respect to the drug, N, N' -diacetylcystine dimethyl ester, 0.5 ml, per injection, of a 400 µg/ml solution was intraperitoneally injected. The injection was conducted three times, 7 days, 4 days and 1 hour before the corneal transplantation. As a control, PBS was intraperitoneally injected. A significant difference in graft survival rate of the corneal transplantation was comparatively detected by the Breslow-Gehan-Wilcoxon test using Kaplan-Meier survival curves. P value of less than 0.05 was defined as significant. NAC (OMe)₂ administration group (N=15) was significantly increased in graft survival rate (P < 0.0001) in comparison to the PBS administration control group (N=10). The high-risk eye graft was rejected by 100% in approximately 3 weeks; whereas in 3 weeks, the rejection was 0%, the cornea of the recipient was stable against inflammation, the transparency was restored, and the graft survival rate of 77% was finally provided.

The timetable of the practice protocol is as follows.
- day 14: suturing of a corneal parenchyma with a nylon yarn for induction of a new blood vessel;
- day 7: intraperitoneal administration of a drug (0.5 ml of 400 µg/ml);
- day 4: intraperitoneal administration of a drug (0.5 ml of 400 µg/ml) ;

day 0 : intraperitoneal administration of a drug (0.5 ml of 400 µg/ml), and corneal transplantation;
day 3: removal of an eyelid suturing yarn (opening of eyelids); and
day 7: removal of a cornea suturing yarn.

The rejection was observed twice a week (till the eighth week).

### (Example 4)

Suppression of skin transplant rejection

Mice, 8 to 10 weeks old, were used. A BALB/c (H-2d) mouse was used as a recipient of transplantation, and a B10.D2 (H-2d) mouse different in minor antigen only was used as a donor. 3 mg of ketamine and 0.0075 mg of xylazine for anesthesia were intraperitoneally administered to the recipient mouse and the transplantation was performed. The skin on the body wall of the donor mouse was transplanted on the thoracic wall (8 x 8 mm) . A gauze having a larger size than the graft was covered thereon, and an antibiotic ointment was applied. The graft was fastened with a cast to complete the operation. The cast was removed after 7 days, and the rejection was then checked every day.

With respect to the drug, N, N'-diacetylcystine diethyl ester, 0.5 ml, per injection, of a 400 µg/ml solution was injected in each of the B10.D2 donor and the BALB/c recipient intraperitoneally and subcutaneously in a site for skin transplantation. The injection was conducted three times, 7 days, 4 days and 1 hour before the skin transplantation. As a control, PBS was intraperitoneally injected. After the skin transplantation, 0.5 ml, per injection, of a 400 µg/ml solution was injected in the BALB/c recipient intraperitoneally and subcutaneously in the graft. The injection timing is day 3, day 7, day 10, day 14, day 17 and day 21 after the skin transplantation. PBS was used as a control. The significant difference in graft survival rate after the skin transplantation was comparatively detected by the Breslow-Gehan-Wilcoxon test using Kaplan-Meier survival curves. P value of less than 0.05 was defined as significant.

The drug administration group (N=8, MST=28.3 ± 1.8 days) was significantly increased in graft survival rate (P < 0.013) in comparison to the PBS administration control group (N=10, MST=14.14 ± 0.47 days).

### (Example 5)

Production of Th1-inductive cytokine IL-12 from reductive macrophages

Mφ was prepared from a knockout mouse deficient in molecules participating in a signal transduction system from a receptor, and a function of redox system was analyzed. Specifically, common Υ chain (Υc) used as a receptor constituting molecule of IL-2, IL-4, IL-7, IL-9 and IL-15 in common and Jak3 as a molecule which presents in the downstream thereof, and transfers a signal from Yc were used as target molecules. The production was performed in the same manner as in Example 2. JAK3 knockout mice were divided into three groups. A control group was a group in which mice were caused to freely drink usual tap water, an NAC group was a group in which mice were caused to freely drink tap water containing 1 mg/ml of NAC, and a GSH-OEt group was a group in which mice was caused to freely drink tap water containing 1 mg/ml of GSH-OEt. The bleeding was continued under SPF conditions for 24 days, and peritoneal exudate adhered cells, namely macrophages were obtained in like manner.

### (Cytokines and stimulator)

A recombinant mouse IFNΥ was made by Genzyme. Recombinant human IL-2 and human IL-6 were made by Ajinomoto Co., Inc. A recombinant human IL-12 was made by Pharmingen.

A product derived from E. coli. 055;B5, as produced by Difco, was used as LPS. As LNT, a preparation thereof produced by Ajinomoto Co., Inc. was used.

### (Quantitative determination of IL-6)

### (Measurement of NO₂⁻ concentration)

### (Detection of intracellular GSH by ACAS)

### (Quantitative determination of IL-12)

These were all performed as in Example 2.

### (Measurement of concentration of Mφ prepared from knockout mice)

Peritoneal cells of knockout mice that underwent the respective treatments were prepared, and the GSH content in cells was analyzed by ACAS using an MCB reagent. In comparison with the control mice of tap water free drinking group, the reduced glutathione content was remarkably increased in mice of both the NAC and GSH-OEt containing tap water free drinking groups, and the image of reductive Mφ induced by the NAC intraperitoneal administration to normal mice was shown.

### (Functions of Mφ prepared from respective groups of treated knockout mice)

Peritoneal cells were prepared from the knockout mice of all the three groups, and stimulated with LPS, IL-2, IFNΥ and a combination thereof to measure the productivities of NO, IL-6 and IL-12. With respect to the NO productivity, almost no productivity is observed in any mice without stimulation. Next, the IL-6 productivity was analyzed. With LPS stimulation, it was 962 pg/ml in the knockout mice, 122 pg/ml in the NAC group, and 82 pg/ml in the GSH-OEt group, respectively. Thus, it was confirmed also from the functional standpoint that macrophages could be converted to reductive macrophages. In consideration of the fact that IL-6 is a main cytokine inducing Th2, it is clearly shown that the biological Th1/Th2 balance can be controlled by oral ingestion of these substances. The results were reversely correlated with the inhibition of the NO production and the recovery pattern with drugs. Then, the IL-12 productivity by the stimulation with LPS and IFNΥ was examined. No productivity was observed at all in the control group. This shows that in the disease animals of JAK3 gene knockout mice used here, the concentration of oxidative macrophages is increased, the humoral immunity or allergic reaction mainly caused by Th2 is increased, and the cellular immunity caused by Th1 is decreased. Meanwhile, the IL-12 productivities of 420 pg/ml and 610 pg/ml were confirmed in the NAC and GSH-OEt administration groups, respectively. These are examples clearly showing that the invention is novel and significant in improving diseases even in the sick animal model.

### (Example 6)

### Difference in IL-12 productivities of reductive and oxidative macrophages

When abnormality occurs in differentiation stage, selection stage and function expressing stage of T cells, biological immune system is broken. From this fact, T cells are considered to play a central part in the immune system. Helper T cells (Th), one of subsets of T cells, are the cells to control immunocompetent cells or inflammatory cells by producing lymphokines. It has been recently insisted that Th is further classified into two types, Th1 and Th2 depending on the type of lymphokines produced and they have different immunological functions, respectively (refer to J. IMMUNOL., vol. 136, PP 2348, 1986). That is, it is considered that Th1 produces IL-2 and IFNΥ and plays a main part in modulation of the cellular immunity, while Th2 produces IL-4, IL-5, IL-6 and IL-10 and plays a main part in modulation of the humoral immunity; the homeostasis of the in-vivo immunomodulation is maintained by the balance of Th1 and Th2. Usually, when the Th1/Th2 balance is inclined to either of Th1 and Th2, the homeostasis is maintained by correcting it. It is however considered that when a state of not correcting the balance is continued for some reason, immune diseases are triggered. Th1 and Th2 are differentiated from a stage of Th0. In the differentiation from Th0 to Th1, IL-12 produced by Mφ is important (refer to IMMUNOLOGY TODAY, vol. 335, PP 14, 1993). In the differentiation from Th0 to Th2, IL-4 produced by NKT cells is important (refer to J. EXP. MEDICINE, vol. 179, PP 1285, 1994).

In Examples above, it is clear that the difference in redox state of Mφ leads to the difference in function of Mφ. In Mφ, there are two types of Mφ, oxidative Mφ and reductive Mφ depending on the difference in GSH content, and these are different in NO or IL-6 production pattern. Main production cells of IL-12, a key molecule that induces differentiation from Th0 to Th1 and controls the Th1/Th2 balance are considered to be Mφ. The present inventors have found that IL-12 is produced only from reductive Mφ. On the basis of this finding obtained in the invention, it is indicated that the redox state of Mφ controls the Th1/Th2 balance, and how useful the invention is for the disease diagnosis of the transplant rejection is described.

### (IL-12 is produced from reductive Mφ)

Mφ prepared by intraperitoneally injecting LNT is reductive Mφ having a high GSH content, and Mφ prepared by intraperitoneally injecting LPS is oxidative Mφ having a low GSH content. It was examined whether or not there was a difference in IL-12 productivity between LNT derived Mφ and LPS derived Mφ. By stimulation with LPS and IFNΥ, the remarkable IL-12 productivity (1,312 pg/ml) was observed in LNT derived Mφ, whereas no productivity was observed in LPS derived Mφ and control resident Mφ. Then, the same analysis was conducted using Mφ prepared by intraperitoneally injecting substances of changing the GSH content in cells. With respect to Mφ prepared by injecting GSH-OEt as a substance of increasing the GSH content in cells and Mφ prepared by injecting BSO and DEM as substances of decreasing the same, IL-12 was produced by stimulation with LPS and IFNY only in Mφ derived from mice administered with GSH-OEt (3, 570 pg/ml). These results reveal that IL-12 is produced only in reductive Mφ having the high GSH content in cells.

### (The IL-12 production from reductive Mφ is inhibited by reducing the GSH content in cells)

It was shown that IL-12 was produced only in reductive Mφ having the high GSH content in cells. It was examined whether or not this production was inhibited by converting Mφ to oxidative Mφ. That is, it was analyzed whether the IL-12 production was inhibited by stimulating the Mφ induced by LNT with DEM or BCNU. As a result, it was found that the IL-12 production (828 pg/ml) from the Mφ induced by LNT was completely inhibited (0 pg/ml) by addition of DEM or BCNU. That is, it was suggested that the IL-12 production was inhibited by exhausting reduced glutathione in cells and converting reductive Mφ to oxidative Mφ.

### Effects of the Invention

The invention enables treatment, improvement and/or prevention of diseases accompanied by rejection in transplantation of human organs such as human liver, heart, lung, kidney, skin, cornea and corneal epithelium (corneal endothelium, epithelial cells of cornea and the like) (including transplantation of parts thereof) and stem cell transplantation of tissues (method for suppressing the transplant rejection), and provides a transplant rejection suppressor quite effective for this purpose, and a use of the foregoing active ingredient (effective ingredient) for this purpose (for production of a transplant rejection suppressor or the like). Further, the invention can provide such a transplant rejection suppressor in the form of pharmaceuticals (including oral preparations, infusions, eye drops and the like), the use thereof, or foods, drinks, nutriments and the like containing this transplant rejection suppressor.

Accordingly, the invention is quite useful in many industrial fields of, especially, medical cares, pharmaceuticals, foods and the like.

## Claims

1. A transplant rejection suppressor, **characterized by** comprising a substance having a function of decreasing a reduced glutathione content in any cells of macrophages, monocytes and dendric cells as an active ingredient.

2. The transplant rejection suppressor as claimed in claim 1, wherein the substance having a function of decreasing a reduced glutathione content is more than one of those decrease the reduced glutathione content in macrophage cells by at least 30% when incubated in vitro with macrophages for from 1 to 24 hours.

3. The transplant rejection suppressor as claimed in claim 1 or 2, wherein the substance having a function of decreasing a reduced glutathione content in any cells of macrophages, monocytes and dendric cells is at least one selected from the group consisting of low-molecular weight compounds such as N, N'-diacylcystine, its esters, busulfan (BCNU), L-S, R-buthionine sulfoximine (BSO), its derivatives and maleic acid diesters.

4. The transplant rejection suppressor as claimed in claim 1 or 2, which is an organ transplant rejection suppressor.

5. The transplant rejection suppressor as claimed in claim 4, wherein the organ is any of liver, lung, kidney, liver, heart, skin, cornea, corneal epithelium and the like,
provided the organ to be transplanted may be a part thereof.

6. The transplant rejection suppressor as claimed in claim 1, wherein the rejection is mainly rejection to a minor antigen.

7. The transplant rejection suppressor as claimed in claim 4 or 5, wherein the organ is cornea, corneal epithelium or the like, and the rejection is mainly rejection to a minor antigen.

8. The transplant rejection suppressor as claimed in any of claims 1 to 7, which is in a form of pharmaceutical (s) .

9. The transplant rejection suppressor as claimed in claim 8, wherein the pharmaceuticals take the form of any of oral drugs, eye drops and infusions.

10. Food, drink or nutriment preparation, **characterized by** containing the transplant rejection suppressor as claimed in any of claims 1 to 7.

11. A method of suppressing transplant rejection, **characterized by** ingesting or administering in vivo a substance having a function of decreasing a reduced glutathione content in any cells of macrophages, monocytes and dendric cells.

12. The method as claimed in claim 11, wherein the ingestion or administration form is in a form of the transplant rejection suppressor as claimed in any of claims 1 to 7.

13. The method as claimed in claim 11 or 12, wherein the ingestion or administration form is in a form of any of pharmaceuticals, foods, drinks and nutriments.

14. A use of a substance having a function of decreasing a reduced glutathione content in any types of cells which are macrophages, monocytes and dendric cells for a transplant rejection suppressor.

15. The use as claimed in claim 14, wherein the transplant rejection suppressor is in a form of pharmaceutical(s), or in a form used in any of foods, drinks and nutriments.

16. The use as claimed in claim 14, wherein the transplant rejection suppressor is the transplant rejection suppressor as claimed in any of claims 1 to 7.
